# EUROPEAN PATENT SPECIFICATION

(11) **EP 3 267 974 B1**
(45) Date of publication and mention of the grant of the patent: **26.12.2018**
(21) Application number: 16716259.3
(22) Date of filing: 09.03.2016
(51) Int. Cl.: A61K 9/00, A61K 31/4168, A61K 31/437, A61K 38/00, A61P 3/10, A61P 9/12, A61P 25/00, A61P 25/04, A61P 25/20, A61P 25/22, A61P 29/00

(54) **A TRANSDERMAL DRUG ADMINISTRATION DEVICE**
TRANSDERMALE WIRKSTOFFFREISETZUNGSVORRICHTUNG
DISPOSITIF D'ADMINISTRATION DE MÉDICAMENT PAR VOIE TRANSDERMIQUE

(30) Priority: 09.03.2015 GB 201503961
(43) Date of publication of application: 17.01.2018
(73) Proprietor: Emplicure AB, 754 50 Uppsala (SE)
(72) Inventor: BREDENBERG, Susanne, 754 50 Uppsala (SE); CAI, Bing, 754 50 Uppsala (SE); ENGQVIST, Håkan, 754 50 Uppsala (SE); XIA, Wei, 754 50 Uppsala (SE)
(74) Representative: Potter Clarkson LLP
(86) International application number: PCT/GB2016/050640
(87) International publication number: WO 2016/142705

(56) References cited:
- EP-A1- 2 823 850
- WO-A1-2007/095859
- WO-A1-2009/094394
- WO-A1-2009/113856
- WO-A2-2012/032337
- BING CAI ET AL: "Self-setting bioceramic microscopic protrusions for transdermal drug delivery", J. MATER. CHEM. B, vol. 2, no. 36, 1 July 2014 (2014-07-01), pages 5992-5998, XP002758798,

## Description

### Field of the Invention

The invention relates to a new transdermal drug administration device including a pharmaceutical composition that provides for the controlled release of active ingredients, such as pain and/or sedative agents, for transdermal administration.

### Background

The listing or discussion of an apparently prior-published document in this specification should not necessarily be taken as an acknowledgement that the document is part of the state of the art or common general knowledge.

Biodegradable microneedles represent the most widely studied microneedles to achieve controlled transdermal drug release. Microneedles made of biodegradable materials have generally higher drug payload and no potentially biohazardous waste after use. Most biodegradable microneedles are made of water-soluble polymers, which can dissolve and release the drug molecules after contact with the interstitial fluid in the skin (Sullivan, S.P., et al., Nat. Med., 2010. 16(8): p. 915-20; Lee, J.W., et al., Biomaterials, 2008. 29(13): p. 2113-2124). However, the polymer materials used to form the microneedles have challenges in their mechanical strength, stability and storage conditions. Microneedles based on bioceramics are also known (Theiss, F., et al., Biomaterials, 2005. 26(21): p. 4383-4394). Despite their good biodegradability and resorbability as shown in some published *in vivo* studies, bioceramics generally have higher mechanical strength and better stability at high temperature and humidity than most polymers. The ability of certain ceramics to be moulded and cured *via* micromoulding is disclosed in Cai, B., et al., Journal of Materials Chemistry B, 2014. 2(36): p. 5992-5998. Observations using microCT have suggested that bioceramic microneedles have sufficient mechanical strength to pierce the skin.

Ceramics are becoming increasingly useful to the medical world, in view of the fact they are durable and stable enough to withstand the corrosive effect of body fluids.

For example, surgeons use bioceramic materials for repair and replacement of human hips, knees, and other body parts. Ceramics also are being used to replace diseased heart valves. When used in the human body as implants or even as coatings for metal replacements, ceramic materials can stimulate bone growth, promote tissue formation and provide protection from the immune system. Dental applications include the use of ceramics for tooth replacement implants and braces.

Ceramics are also known to be of potential use as fillers or carriers in controlled-release pharmaceutical formulations. See, for example, EP 947 489 A, US 5,318,779, WO 2008/118096, Lasserre and Bajpai, Critical Reviews in Therapeutic Drug Carrier Systems, 15, 1 (1998), Byrne and Deasy, Journal of Microencapsulation, 22, 423 (2005) and Levis and Deasy, Int. J. Pharm., 253, 145 (2003).

In particular, Rimoli et al, J. Biomed. Mater. Res., 87A, 156 (2008), US patent application 2006/0165787 and international patent applications WO 2006/096544, WO 2006/017336 and WO 2008/142572 all disclose various ceramic substances for controlled release of active ingredients, with the latter two documents being directed in whole or in part to opioid analgesics, with the abuse-resistance being imparted by the ceramic structures' mechanical strength.

A composite material having a beneficial agent associated with at least a portion of a high surface area component so as to increase the bioavailability and/or activity of the beneficial agent is disclosed in WO 02/13787. The high surface area component may be formed from a material having a hardness that is greater than the hardness of the beneficial agent, and may be formed from metal oxides, metal nitrides, metal carbides, metal phosphates, carbonaceous materials, ceramic materials and mixtures thereof. The beneficial agent may be associated with the high surface area component by means of spraying, brushing, rolling, dip coating, powder coating, misting and/or chemical vapour deposition.

Various methods of enhancing drug delivery by transdermal administration are described by Banga in Expert Opin. Drug Deliv., 6, 343 (2009), including direct coating onto microneedles and administration *via* hollow microneedles. See also international patent application WO 03/090729 and WO 2009/113856, US patent No. US 6,334,856 and US patent application No. US 2009/0200262.

An interface for a transdermal drug administration device is disclosed in US 2007/0123837. The interface may be provided in the form of a flat plate including two-dimensionally arranged projections, capable of piercing the skin, and a plurality of openings, capable of delivering a drug, respectively arranged in correspondence with the projections. The projections may be conical or pyramidal in shape and the flat plate and projections may be formed from a metal, an alloy or a ceramic. In use, in a transdermal drug administration device for example, a drug in liquid form may be held in a drug-containing layer above the flat plate. When the flat plate is pressed against the skin, the plurality of projections pierce the skin and the drug is transferred from the drug-containing layer, *via* the plurality of openings provided in correspondence with the projections, through the holes formed in the skin.

A device for delivering bioactive agents through the skin is also disclosed in international patent application no. WO 03/092785. The device includes a plurality of skin-piercing members and a porous calcium phosphate coating adapted as a carrier and provided on at least part of the skin-piercing members. The coating includes at least one bioactive agent and the skin-piercing members may be formed from metals, ceramics, plastics, semiconductors or composite materials.

Each of these documents refers to the possibility of loading and/or combining an active ingredient with a delivery device, either by means of a separate drug-containing layer provided in combination with the device or a coating applied to the device.

Due to the elasticity and toughness of the skin, needle arrays can encounter the "bed of nails" effect, in which the force applied by the user is distributed across all of the needles, reducing the efficiency of insertion. This can lead to insufficient and inconsistent drug delivery and wastage of drugs.

Methods of manufacturing polymer microneedles with flexible and water-soluble substrates, made of polyvinylpyrrolidone / polyvinylacetate, are disclosed in Moga, K.A., et al., Advanced Materials, 2013. 25(36): p. 5060-5066. However, the embossing process accompanied by high temperature might denature thermally sensitive drugs and lead to unreliable attachment to the ceramic microneedles.

A transdermal administration device with a layer of water-swelling polymers is disclosed in US patent No. 5,250,023. The device can be adhered to the skin and the needles with diameter less than 400µm and shorter than 2mm would be inserted into skin by electrical generated compression power. After the delivery, the force initiated by the swelling of polymer layer would withdraw the needles from the skin thus allowing only a temporary penetration of the needles into the skin.

Bing Cai et al, J. Mater. Chem. B, 2014, vol. 2, no. 36, 5992-5998, discloses bioceramic microneedles (made of calcium sulphate or calcium phosphate) for their use in transdermal drug delivery. Zolpidem is used as the model drug. The microneedles are not disclosed to be attached to any substrate.

The applicant has unexpectedly found that a combination of a drug delivery element (based on a porous solid) and a flexible, water-swelling substrate may be able to address some of the problems associated with the prior art devices. The devices of the invention disclosed herein are useful in *inter alia* delivering a variety of drugs (including opioid drugs) to patients.

Opioids are widely used in medicine as analgesics, for example in the treatment of patients with severe pain, chronic pain or to manage pain after surgery. Indeed, it is presently accepted that, in the palliation of more severe pain, no more effective therapeutic agents exist.

The term "opioid" is typically used to describe a drug that activates opioid receptors, which are found in the brain, the spinal chord and the gut. Three classes of opioids exist:
(a) naturally-occurring opium alkaloids. These include morphine and codeine;
(b) compounds that are similar in their chemical structure to the naturally occurring alkaloids. These so-called semi-synthetics are produced by chemical modification of the latter and include the likes of diamorphine (heroin), oxycodone and hydrocodone; and
(c) truly synthetic compounds such as fentanyl and methadone. Such compounds may be completely different in terms of their chemical structures to the naturally-occurring compounds.

Of the three major classes of opioid receptors (µ, κ and δ), opioids' analgesic and sedative properties mainly derive from agonism at the µ receptor.

Opioid analgesics are used to treat severe, chronic cancer pain, often in combination with non-steroid anti-inflammatory drugs (NSAIDs), as well as acute pain (e.g. during recovery from surgery and breakthrough pain). Further, their use is increasing in the management of chronic, non-malignant pain.

### Disclosure of the Invention

The invention is defined by the appended claims.

According to the invention there is provided a transdermal drug administration device comprising a drug delivery element attached to a water-swellable substrate comprising one or more organic polymers, wherein the drug delivery element defines a contact surface for location, in use, against a patient's skin, further wherein the drug delivery element comprises an active pharmaceutical ingredient and a porous solid material, wherein the porous solid material is based on one or more ceramic materials or one or more geopolymeric materials.

We have advantageously found that the transdermal drug administration devices of the invention provide for tunable, controlled and uniform release of active ingredient into a patient through the skin. The combination of the water-swellable substrate and porous solid also allows the device to be manufactured under "mild" conditions, and facilitates control of the attachment and detachment of the water-swellable substrate from the porous solid.

The term "porous solid" refers to a substance that is a solid, continuous network containing pores. According to the invention, the porous solid is based on one or more ceramic materials or one or more geopolymeric materials.

The drug delivery element, which comprises an active pharmaceutical ingredient and a porous solid material, may be formed directly from a material that inherently possesses a high mechanical strength or it may be formed as a consequence of a chemical reaction between one or more precursor substances or materials, so forming the three-dimensional network *in situ.* In this respect, the network may be designed to be inert in the following way. General physico-chemical stability under normal storage conditions, including temperatures of between about minus 80 and about plus 50°C (preferably between about 0 and about 40°C and more preferably room temperatures, such as about 15 to about 30°C), pressures of between about 0.1 and about 2 bars (preferably at atmospheric pressure), relative humidities of between about 5 and about 95% (preferably about 10 to about 75%), and/or exposure to about 460 lux of UV/visible light, for prolonged periods (i.e. greater than or equal to six months). Under such conditions, carrier material networks as described herein may be found to be less than about 5%, such as less than about 1% chemically degraded/decomposed, as above.

In this respect, by network of "high mechanical strength" we also include that the structure of the porous solid material maintains its overall integrity (e.g. shape, size, porosity, etc) when a force of about 1 kg-force/cm² (9 newtons/cm²), such as about 5 kg-force/cm² (45 newtons/cm²), such as about 7.5 kg-force/cm², e.g. about 10.0 kg-force/cm², preferably about 15 kg-force/cm², more preferably about 20 kg-force/cm², for example about 50 kg-force/cm², especially about 100 kg-force/cm² or even about 125 kg-force/cm² (1125 newtons/cm²) is applied using routine mechanical strength testing techniques known to the skilled person (for example using a so-called "compression test" or "diametral compression test", employing a suitable instrument, such as that produced by Instron (the "Instron Test", in which a specimen is compressed, deformation at various loads is recorded, compressive stress and strain are calculated and plotted as a stress-strain diagram which is used to determine elastic limit, proportional limit, yield point, yield strength and (for some materials) compressive strength)).

In embodiments in which ceramics such as calcium sulphate and/or calcium phosphate are used, the structure of the porous solid material may maintain its overall integrity (e.g. shape, size, porosity, etc) when a generally lower force is applied. This may be, for example, when a force of about 0.1 kg-force/cm² (0.9 newtons/cm²), such as about 0.5 kg-force/cm² (4.5 newtons/cm²), such as about 0.75 kg-force/cm², e.g. about 1.0 kg-force/cm², preferably about 1.5 kg-force/cm², more preferably about 2.0 kg-force/cm², for example about 5.0 kg-force/cm², especially about 10.0 kg-force/cm² or even about 12.5 kg-force/cm² (112.5 newtons/cm²) is applied using routine mechanical strength testing techniques, such as those described above.

According to the invention, the porous solid is be based on one or more ceramic materials or one or more geopolymeric materials. Preferably, the porous solid is based on one or more ceramic materials (e.g. a bioceramic material).

The term "ceramic" will be understood to include compounds formed between metallic and nonmetallic elements, frequently oxides, nitrides and carbides that are formed and/or processable by some form of curing process, which often includes the action of heat. In this respect, clay materials, cement and glasses are included within the definition of ceramics (Callister, "Material Science and Engineering, An Introduction" John Wiley & Sons, 7th edition (2007)). Preferred ceramic materials are ceramic materials that are biocompatible (i.e. so-called "bioceramic materials").

Ceramics may comprise chemically bonded ceramics (non-hydrated, partly hydrated or fully hydrated ceramics, or combinations thereof). Non-limiting examples of chemically bonded ceramics systems include calcium phosphates, calcium sulphates, calcium carbonates, calcium silicates, calcium aluminates and magnesium carbonates. Preferred chemical compositions include those based on chemically bonded ceramics, which following hydration of one or more appropriate precursor substances consume a controlled amount of water to form a network. In particular embodiments, the network has a high mechanical strength. The preferred systems available are those based on calcium sulphates, calcium phosphates, calcium silicates, calcium carbonates and magnesium carbonates. For the avoidance of doubt, the porous solid may comprise more than one ceramic material.

In particular embodiments of the invention, the porous solid is based on a ceramic material that is formed from a self-setting ceramic. The use of these and other particular ceramics facilitates the formation of the drug delivery element in such a way that the active pharmaceutical ingredient would not be exposed to harsh conditions (e.g. high temperatures, such as temperatures exceeding 60°C) during said formation. Non-limiting examples of self-setting ceramics include calcium sulphate, calcium phosphate, calcium silicate and calcium aluminate based materials. Particular ceramics that may be mentioned in this respect include alpha-tricalcium phosphate, hemihydrate calcium sulphate, CaOAl₂O₃, CaO(SiO₂)₃, CaO(SiO₂)₂, and the like

It is preferred that the ceramic material that is employed is based upon a sulfate, such as a calcium sulfate or a phosphate such as a calcium phosphate. Particular examples of such substances include calcium sulfate hemihydrate (end product calcium sulphate) and acidic calcium phosphate (brushite). However, the porous solid may also be made from an oxide and/or a double oxide, and/or a nitride, and/or a carbide, and/or a silicate and/or an aluminate of any of the elements silicon, aluminium, carbon, boron, titanium, zirconium, tantalum, scandium, cerium, yttrium or combinations thereof. Such materials may be crystalline or amorphous.

Non-limiting examples of aluminium silicates and aluminium silicate hydrates that may be used to form the porous solid in the present invention include kaolin, dickite, halloysite, nacrite, ceolite, illite or combinations thereof, particularly halloysite. The grain size of the ceramic material (e.g. aluminium silicate) may be below about 500 µm, preferably below about 100 µm, and particularly below about 20 µm, as measured by laser diffraction in the volume average mode (e.g. Malvern master size). The grains may be of any shape (e.g. spherical, rounded, needle, plates, etc.).

The mean grain size of any ceramic precursor powder particles may be below about 100 µm, preferably between about 1 µm and about 20 µm. This is to enhance hydration. Such precursor material may be transformed into a nano-size microstructure during hydration. This reaction involves dissolution of the precursor material and repeated subsequent precipitation of nano-size hydrates in the water (solution) and upon remaining non-hydrated precursor material. This reaction favourably continues until precursor materials have been transformed and/or until a pre-selected porosity determined by partial hydration using the time and temperature, as well as the H₂O in liquid and/or humidity, is measured.

In other (e.g. preferred) embodiments of the invention, the porous solid may be based on one or more geopolymer materials.

The term "geopolymer" will be understood by those skilled in the art to include or mean any material selected from the class of synthetic or natural aluminosilicate materials which may be formed by reaction of an aluminosilicate precursor substance (preferably in the form of a powder) with an aqueous alkaline liquid (e.g. solution), preferably in the presence of a source of silica. For the avoidance of doubt, the porous solid may comprise more than one geopolymer material.

The term "source of silica" will be understood to include any form of a silicon oxide, such as SiO₂, including a silicate. The skilled person will appreciate that silica may be manufactured in several forms, including glass, crystal, gel, aerogel, fumed silica (or pyrogenic silica) and colloidal silica (e.g. Aerosil).

Suitable aluminosilicate precursor substances are typically (but not necessarily) crystalline in their nature and include kaolin, dickite, halloysite, nacrite, zeolites, illite, preferably dehydroxylated zeolite, halloysite or kaolin and, more preferably, metakaolin (i.e. dehydroxylated kaolin). Dehydroxylation (of e.g. kaolin) is preferably performed by calcining (i.e. heating) of hydroxylated aluminosilicate at temperatures above 400°C. For example, metakaolin may be prepared as described by Stevenson and Sagoe-Crentsil in J. Mater. Sci., 40, 2023 (2005) and Zoulgami et al in Eur. Phys J. AP, 19, 173 (2002), and/or as described hereinafter. Dehydroxylated aluminosilicate may also be manufactured by condensation of a source of silica and a vapour comprising a source of alumina (e.g. Al₂O₃).

Precursor substances may also be manufactured using sol-gel methods, typically leading to nanometer sized amorphous powder (or partly crystalline) precursors of aluminosilicate, as described in Zheng et al in J. Materials Science, 44, 3991-3996 (2009). This results in a finer microstructure of the hardened material. (Such as sol-gel route may also be used in the manufacture of precursor substances for the chemically bonded ceramic materials hereinbefore described.)

If provided in the form of a powder, the mean grain size of the aluminosilicate precursor particles are below about 500 µm, preferably below about 100 µm, more preferred below about 30 µm.

In the formation of geopolymer materials, such precursor substances may be dissolved in an aqueous alkaline solution, for example with a pH value of at least about 12, such as at least about 13. Suitable sources of hydroxide ions include strong inorganic bases, such as alkali or alkaline earth metal (e.g. Ba, Mg or, more preferably, Ca or, especially Na or K, or combinations thereof) hydroxides (e.g. sodium hydroxide). The molar ratio of metal cation to water can vary between about 1:100 and about 10:1, preferably between about 1:20 and about 1:2.

A source of silica (e.g. a silicate, such as SiO₂) is preferably added to the reaction mixture by some means. For example, the aqueous alkaline liquid may comprise SiO₂, forming what is often referred to as waterglass, i.e. a sodium silicate solution. In such instances, the amount of SiO₂ to water in the liquid is preferably up to about 2:1, more preferably up to about 1:1, and most preferably up to about 1:2. The aqueous liquid may also optionally contain sodium aluminate.

Silicate (and/or alumina) may alternatively be added to the optionally powdered aluminosilicate precursor, preferably as fume silica (microsilica; AEROSIL® silica). The amount that may be added is preferably up to about 30 wt%, more preferably up to about 5 wt.% of the aluminosilicate precursor.

The presence of free hydroxide ions in this intermediate alkaline mixture causes aluminium and silicon atoms from the source material(s) to be dissolved. The geopolymer materials may then be formed by allowing the resultant mixture to set (cure or harden), during which process the aluminium and silicon atoms from the source materials reorientate to form a hard (and at least largely) amorphous geopolymeric material. Curing may be performed at room temperature, at elevated temperature or at reduced temperature, for example at around or just above ambient temperature (e.g. between about 20°C and about 90°C, such as around 40°C). The hardening may also be performed in any atmosphere, humidity or pressure (e.g. under vacuum or otherwise). The resultant inorganic polymer network is in general a highly-coordinated 3-dimensional aluminosilicate gel, with the negative charges on tetrahedral Al³⁺ sites charge-balanced by alkali metal cations.

In this respect, a geopolymer-based porous solid may be formed by mixing a powder comprising the aluminosilicate precursor and an aqueous liquid (e.g. solution) comprising water, a source of hydroxide ions as described hereinbefore and the source of silica (e.g. silicate), to form a paste. The ratio of the liquid to the powder is preferably between about 0.2 and about 20 (w/w), more preferably between about 0.3 and about 10 (w/w). Calcium silicate and calcium aluminate may also be added to the aluminosilicate precursor component.

If the porous solid is formed by way of a chemical reaction (e.g. polymerisation, or as described hereinbefore for geopolymers), active ingredient may be co-mixed with a precursor mixture comprising relevant reactants and thereafter located within pores or voids that are formed during formation of the porous solid (i.e. the three-dimensional carrier material network) itself. Although it is not essential in all cases, it may be that, in some cases, it is necessary to include a porogenic material as part of the reaction mixture in order to assist in the formation of pores within the final carrier material network, within which active pharmaceutical ingredient is co-formedly interspersed. Porogenic materials include, for example, oils, liquids (e.g. water), sugars, mannitol etc.

In an embodiment of the invention, the active pharmaceutical ingredient is predominantly located within the pores of the porous solid. By this, it is meant that at least 50% by weight of the total amount of the active pharmaceutical ingredient present in the drug delivery element is located within the pores of the porous solid. In particular embodiments, at least 70%, e.g. at least 80% (preferably at least 90%) of the active pharmaceutical ingredient present in the drug delivery element is located within the pores of the porous solid.

In a particular embodiment of the invention, the active pharmaceutical ingredient is predominantly located on the outer surface of the porous solid. By this, it is meant that at least 50% by weight of the total amount of the active pharmaceutical ingredient present in the drug delivery element is located on the outer surface of the porous solid (i.e. on the surface which is intended to come into contact with the skin of the patient). In particular embodiments, at least 70%, e.g. at least 80% (preferably at least 90%) of the active pharmaceutical ingredient present in the drug delivery element is located on the outer surface of the porous solid. The active pharmaceutical ingredient may be combined with the drug delivery element by means of spraying, brushing, rolling, dip coating, powder coating, misting and/or chemical vapour deposition.

The composition may further include a film forming agent co-formedly interspersed within the pores of the network. When used herein, the term "film-forming agent" refers to a substance that is capable of forming a film over (or within), or coating over, another substance or surface (which may be in particulate form).

The use of a film forming agent improves the tamper resistance of the transdermal drug administration device and may also further advantageously increase the mechanical strength of the composition. These features provide advantages associated with the prevention of dose dumping and potential misuse or drug abuse by *ex vivo* extraction of the active pharmaceutical ingredient, when the latter comprises an opioid analgesic or other compound with a risk of misuse/abuse.

The transdermal drug administration device may further comprise one or more commonly-employed pharmaceutical excipients. Suitable excipients include inactive substances that are typically used as a carrier for the active pharmaceutical ingredients in medications. Suitable excipients also include those that are employed in the pharmaceutical arts to bulk up drug delivery systems that employ very potent active pharmaceutical ingredients, to allow for convenient and accurate dosing. Alternatively, excipients may also be employed to aid in the handling of the active pharmaceutical ingredient concerned.

In this respect, pharmaceutically-acceptable excipients include filler particles, by which we include particles that do not take part in any chemical reaction during which a composition is formed. Such filler particles may be added as ballast and/or may provide the composition with functionality.

The composition may also optionally contain bulking agents, porogens, pH modifiers, dispersion agents or gelating agents to control the rheology or the amount of liquid in the porous solid. The total amount of such excipients is limited to about 20 wt% of the total weight of the porous solid (or the materials from which it is formed). Non-limiting examples of such excipients include polycarboxylic acids, cellulose, polyvinylalcohol, polyvinylpyrrolidone, starch, nitrilotriacetic acid (NTA), polyacrylic acids, PEG, glycerol, sorbitol, mannitol and combinations thereof.

Additional pharmaceutically-acceptable excipients include carbohydrates and inorganic salts such as sodium chloride, calcium phosphates, calcium carbonate, calcium silicate and calcium aluminate. In the case of porous solids based on geopolymers, such additional materials are preferably added to the aluminosilicate precursor component. Compositions of the invention may also comprise disintegrant and/or superdisintegrant materials. Such materials may be presented, at least in part, within the porous solid material.

The disintegrant or "disintegrating agent" that may be employed may be defined as any material that is capable of accelerating to a measurable degree the disintegration/dispersion of the transdermal drug administration device of the invention. The disintegrant may thus provide for an *in vitro* disintegration time of about 30 seconds or less, as measured according to e.g. the standard United States Pharmacopeia (USP) disintegration test method (see *FDA Guidance for Industry: Orally Disintegrating Tablets;* December 2008). This may be achieved, for example, by the material being capable of swelling, wicking and/or deformation when placed in contact with water and/or mucous (e.g. saliva), thus causing tablet formulations to disintegrate when so wetted.

Suitable disintegrants (as defined in, for example, Rowe et al, Handbook of Pharmaceutical Excipients, 6th ed. (2009)) include cellulose derivatives such as hydroxypropyl cellulose (HPC), low substituted HPC, methyl cellulose, ethyl hydroxyethyl cellulose, carboxymethyl cellulose calcium, carboxymethyl cellulose sodium, microcrystalline cellulose, modified cellulose gum; starch derivatives such as moderately cross-linked starch, modified starch, hydroxylpropyl starch and pregelatinized starch; and other disintegrants such as calcium alginate, sodium alginate, alginic acid, chitosan, docusate sodium, guar gum, magnesium aluminium silicate, polacrilin potassium and polyvinylpyrrolidone. Combinations of two or more disintegrants may be used.

Preferred disintegrants include so-called "superdisintergrants" (as defined in, for example, Mohanachandran et al, International Journal of Pharmaceutical Sciences Review and Research, 6, 105 (2011)), such as cross-linked polyvinylpyrrolidone, sodium starch glycolate and croscarmellose sodium. Combinations of two or more superdisintegrants may be used.

Disintegrants may also be combined with superdisintegrants in the transdermal drug administration devices of the invention.

In preferred embodiments, the disintegrants and/or superdisintegrants may be located primarily within the drug delivery element (i.e. together with the porous solid material). In such embodiments, the disintegrants and/or superdisintegrants are preferably employed in an (e.g. total) amount of between 0.5 and 15% by weight based upon the total weight of the drug delivery element. A preferred range is from about 0.1 to about 5%, such as from about 0.2 to about 3% (e.g. about 0.5%, such as about 2%) by weight.

If employed in particulate form, particles of disintegrants and/or superdisintegrants may be presented with a particle size (weight and/or volume based average or mean diameter, *vide supra*) of between about 0.1 and about 100 µm (e.g. about 1 and about 50 µm).

Alternatively, disintegrants and/or superdisintegrants may also be present as a constituent in composite excipients. Composite excipients may be defined as co-processed excipient mixtures. Examples of composite excipients comprising superdisintegrants are Parteck® ODT, Ludipress® and Prosolv® EASYtab.

It is particularly preferred that the disintegrants and/or superdisintegrants are predominantly contained (i.e. at least 80% of the disintegrants and/or superdisintegrants are contained) within the drug delivery element.

As defined herein, the drug delivery element defines a contact surface for location, in use, against a patient's skin, and includes the combination of the porous solid and the active pharmaceutical ingredient.

As defined herein, the drug delivery element defines a contact surface for location, in use, against a patient's skin and includes a combination of a porous solid and an active pharmaceutical ingredient. Accordingly, it is not essential that the drug delivery element is placed in direct contact with the skin. Indeed, the drug delivery element may be coated with a coating material (e.g. a thin, porous film or hydrophilic or hydrophobic chemical substances, such as surface active molecules, e.g. silicones or fluoroalkyl materials).

The drug delivery element of the drug administration device according to the invention may take several forms, provided that it defines a contact surface for location, in use, against a patient's skin.

In one embodiment, the composition that is used to form the drug delivery element may be moulded during formation into one or more homogeneous layers (e.g. in the form of one or more uniform layers, elements, plates or disks) that may be flat and/or thin defining a drug delivery element containing the active pharmaceutical ingredient and the porous solid. Typical dimensions for a single drug delivery element to be applied to the skin may be in the range of between about 2 cm (e.g. about 5 cm) and about 10 cm by about 2 cm (e.g. about 5 cm) and about 10 cm. Preferred size ranges for single elements are about 5 cm by about 5 cm, such as about 2 cm by about 2 cm, with a thickness of up to about 1 cm, preferably up to about 0.5 cm, such as up to about 0.02 cm. Any of the aforementioned dimensions may be used in combination. Furthermore, multiple elements of the same or different dimensions (e.g. smaller elements of about 1 mm by about 1 mm) may be applied to the skin at the same time to make a "mosaic" pattern of elements.

In such embodiments, the homogeneous layer may be moulded to define a substantially flat contact surface for location, in use, against a patient's skin (in either direct or indirect contact as described hereinbefore).

The term "substantially flat contact surface" will be understood to include a flat contact surface that excludes any pre-formed protrusions and includes only undulations or variations resulting from the moulding process.

In preferred embodiments, the drug delivery element may comprise an array of microscopic protrusions for location, in use, against a patient's skin. This array may be formed by moulding the drug delivery element during its formation. Alternatively, the array of microscopic protrusions may be formed by etching a sample of the drug delivery element.

In one embodiment, the homogeneous layer may be moulded to define a contact surface including an array of microscopic protrusions for location, in use, against a patient's skin.

The term "array" refers to any arrangement of said microscopic protrusions on the surface of the drug delivery element. In a preferred embodiment, substantially all of the microscopic protrusions are located on a single surface of the drug delivery element. It is not necessary for the microscopic protrusions to be arranged in an ordered way.

Typically, the drug delivery element will comprise an array of microscopic protrusions in which the surface density of microscopic protrusions on the drug delivery element ranges from about 10 to about 10,000 microscopic protrusions per square centimetre. Preferred surface densities are from about 20 to about 2000 (e.g. from about 50 to about 1000) microscopic protrusions per square centimetre.

The "microscopic protrusions" may be provided in the form of any shape that has a base and one or more sloping sides to define (e.g. in the case of more than one side to meet generally centrally at) an apex (i.e. point or ridge, which may be rounded), for example conical or pyramidal protrusions or conical protrusions. Such protrusions may be of about 10 µm to about 1500 µm in height and have a width at their lower bases of about 0.1 µm to about 400 µm. In embodiments of the invention, the microscopic protrusions may have an aspect ratio ranging from about 1 to about 9. The most appropriate aspect ratio may depend on the choice of material used to form the drug delivery element. For example, if a ceramic material is used, a preferred aspect ratio would be from about 1 to about 4 (such as from about 2 to about 3). For polymer-based microneedles, a preferred aspect ratio would be from about 1 to about 5.

The provision of microscopic protrusions increases the surface area of the contact surface of the drug element available for location against a patient's skin and thereby increases the size (i.e. the contact surface area) of the drug reservoir available for administration *via* the patient's skin. This improves the transport of the active pharmaceutical ingredient from the drug delivery element *via* pores in the skin barrier so as to facilitate absorption of the active pharmaceutical ingredient through the skin barrier. It thus improves the efficiency of the drug delivery element in administering the active pharmaceutical ingredient to the patient. The use of such microscopic protrusions is advantageous in the treatment of e.g. chronic disorders in which the ongoing administration of an active pharmaceutical ingredient is required.

The provision of microscopic protrusions also enables the drug delivery element to pierce the outer layers of the skin of the patient, thereby facilitating the flow of the active pharmaceutical agent through the skin barrier into the patient.

Other shapes may be moulded into the contact surface(s) of the drug delivery element in order to increase hydrophobicity or hydrophilicity of at least part of the resultant surface (with or without the employment of surface active molecules). The drug delivery element may thus make use of the so-called "lotus effect", in which the contact angle of certain microscopic protrusion(s) at the surface is high enough (e.g. >90°) to be hydrophobic and/or low enough (e.g. <90°) to be hydrophilic. The moulded structure may thus be designed so that the surface of the drug delivery element is capable of channelling moisture from one part to another, for example any part of the drug delivery element where there are pores comprising active ingredient.

It is most preferred that the drug delivery element comprises an array of microscopic protrusions wherein the microscopic protrusions are not in direct contact with each other (e.g. they are not linked *via* a layer composed of the same porous solid material) but are instead linked together *via* the substrate (i.e. the backing layer). For example, the porous solid from which the drug delivery element is formed may only be present in the transdermal drug administration device within the microscopic protrusions, whereas the regions of the contact surface between the microscopic protrusions are formed from the substrate (i.e. the backing layer). In such embodiments, once the substrate is removed, the microscopic protrusion are no longer linked together.

Combinations of the aforementioned microscopic protrusion patterns may be employed in the drug delivery element.

In a further embodiment, the homogeneous layer may be moulded to define an array of micro-needles protruding from the contact surface of the drug delivery element.

The term "micro-needles" will be understood to include sharp protrusions having a length of 4 µm to 700 µm and having a width at their lower bases of 1 µm to 200 µm, which, on placement of a contact surface including an array of micro-needles against a patient's skin, create micron-sized micropores or microchannels in the skin. This facilitates more rapid delivery of active pharmaceutical ingredients, and/or the delivery of larger molecules such as peptides, proteins, antigens and other immunogenic substances (e.g. vaccines), for example, which cannot otherwise penetrate the skin barrier.

The size of the micro-needles moulded so as to protrude from the contact surface of the drug delivery element may be varied depending on the nature of the active pharmaceutical ingredient interspersed in the drug delivery element so as to alter the extent of penetration of the needles into the skin barrier.

The homogeneous layer from which the drug delivery element is formed may be moulded to define an array of solid micro-needles, and may further be moulded to define an array of hollow micro-needles. The use of hollow micro-needles allows the accurate delivery of larger molecules of active pharmaceutical ingredient *via* holes formed in the tips of the micro-needles directly into the micropores or microchannels formed in a patient's skin. Any such holes may have a diameter of between 10 µm and 100 µm.

The use of micro-needles that penetrate a patient's skin is advantageous in the treatment of acute disorders in which a rapid onset of action from an active pharmaceutical ingredient is required. Embodiments that are useful under such circumstances are those which provide for instant release of the active pharmaceutical ingredient upon application of the transdermal drug administration device on the skin of the patient. The creation of micropores or microchannels in the patient's skin accelerates the rate at which drug molecules can be absorbed into the patient's bloodstream when compared with the use of a flat contact surface or a contact surface including a plurality of microscopic protrusions.

In embodiments in which the drug delivery element is provided in the form of a homogeneous layer of the composition, so as to define a substantially flat contact surface or so as to define a contact surface including an array of microscopic protrusions or micro-needles protruding therefrom, the homogeneous layer may be formed by filling a production mould with a wet mass comprising an active pharmaceutical ingredient and a porous solid or precursor(s) thereto, and forming the curing or bonding step mentioned hereinbefore *in situ.*

The mould is chosen to define the desired geometry of the resultant homogeneous layer and the wet mass is preferably chemically hardened (i.e. is hardened or otherwise cured *via* chemical reactions) to form the porous solid. In particular embodiments, the active pharmaceutical ingredient is present during the hardening of the wet mass, and this results in the active pharmaceutical ingredient being co-formedly dispersed in the pores of the hardened solid. In other embodiments, the active pharmaceutical ingredient is introduced into the porous solid after the solid has been formed.

Such moulded elements may be formed by mixing together the porous solid (according to the invention, a ceramic or geopolymeric material), or precursor(s) thereto, and the active substance, along with, or in, a liquid, such as an aqueous solvent (e.g. water), so providing a wet paste, and directly moulding the paste into the desired shape. The paste is preferably moulded into a polymer mould or into polymer coated metal or ceramic mould (e.g. Teflon coating). After moulding, the paste may be hardened (in a preferably warm and moist environment) to the final desired shape. For example, in the case of geopolymer-based carrier materials, aluminosilicate precursor may be reacted together with aqueous alkaline liquid (e.g. solution), preferably in the presence of a source of silica (as hereinbefore described), also in the presence of the active ingredient (and/or other excipients, such as a film-forming agent) as hereinbefore described and curing thereafter performed by allowing the resultant mixture to harden into the required homogeneous layer shape. Alternatively, preformed geopolymer may be reacted together with further aluminosilicate precursor and aqueous alkaline liquid (e.g. solution), in the presence of the active ingredient and optionally a source of silica and curing thereafter performed as described above. In this respect, the mixture may be transferred into moulds and left to set as the homogeneous layer.

In such embodiments, the mould in which the homogeneous layer of composition is formed may form a blister packaging for the drug delivery element, the bottom of the blister forming the negative mould for any microscopic protrusions or micro-needles formed so as to protrude from the contact surface.

Such moulds may be formed by etching (chemical or physical (e.g. by way of a laser)) or known micromechanical techniques, such as soft lithography. Soft lithography is the general name for a number of different nanofabrication techniques in which a master initially is produced on a silicon wafer, for example UV-photolithography. Here, a device layout is printed on a transparency or on a chrome mask, making some areas transparent and others oblique to UV-light. A silicon wafer is then spin-coated with a photo-curable resist, which is exposed to UV-light through the mask. The wafer is then subjected to an etching solution that removes the uncured photoresist to make the master. The master is then used as a mould to cast a negative structure in an elastomer. This elastomer casting is either the end product, or it in turn is used as a mould to make another generation of castings with structures similar to those of the silicon master (see, for example, Madou, Fundamentals of Microfabrication: The Science of Miniaturization, 2nd ed. (2002), Boca Raton: CRC Press. 723 and Weigl et al, Advanced Drug Delivery Reviews (2003) 55, 349-377 for further information).

The term "substrate" refers to a backing layer to which the drug delivery element is attached. Particular substrates that may be mentioned are those provided in the form of a flexible film. It is preferred that the substrate is sufficiently flexible (under ambient conditions) to allow it to be deformed against a patient's skin. That is, the substrate should not be rigid but should instead be pliable so that the user may easily adjust the contours of the delivery device to allow it to substantively match the contours of the area of skin to which the device is to be applied. The use of a flexible substrate in combination with a drug delivery element comprising an array of microscopic protrusions facilitates the delivery of the active ingredient to the patient. The use of a flexible substrate reduces the effort required to penetrate the skin and minimises the "bed of nails" effect which can inhibit effective penetration of the protrusions into the skin. The "bed of nails" effect occurs when a rigid substrate is used. When a rigid substrate is used, some of the microscopic protrusions making up the drug administration device might not penetrate the skin and others might be pulled out with the movement of the substrate. Both of these issues could lead to insufficient and inconsistent drug delivery, and wastage of drugs.

The material that forms the substrate should be water-swellable. That is, the material swells when brought into contact with an aqueous medium, such as the patient's interstitial fluid and/or mucous (e.g. saliva). The use of such substrates allows the backing layer to swell when the transdermal drug administration device is brought into contact with human body tissue due to the moisture that is naturally present (e.g. interstitial fluid in the skin). The term "swells" refers to an increase in the volume of a given material. In particular embodiments, the water-swellable substrates of the present invention are made from materials that are capable of increasing in volume by at least 100% (e.g. at least 200%, such as at least 500%) when brought into contact with water. Particularly preferred water-swellable substrates are those made from materials that are capable of increasing in volume by from 500% to 1000%.

According to the invention, the substrate is a water-swellable substrate (i.e. a water-swellable backing layer).

The substrate is made from one or more organic polymers. Suitable polymers for use as substrates in the transdermal drug administration device of the present invention include fenugreek gum, sesbania gum, cyclodextrin, PVA (polyvinyl alcohol), and particularly silicon rubber, polymethyl methacrylate (PMMA), polydimethyl siloxane (PDMS), polyethylene (PE), polypropylene (PP), parylene, polyvinylpyrrolidone, polyvinylacetate, alginate (e.g. ammonium alginate), chitosan, gelatin, polyvinyl alcohol copolymers, polyacrylamide, carboxymethylcellulose, polyvinylimine, polyacrylate and karaya gum. Preferred polymers include fenugreek gum, sesbania gum, cyclodextrin, PVA, and particularly gelatin, polyvinyl alcohol copolymers, polyacrylamide, carboxymethylcellulose, polyvinylimine, polyacrylate, alginate and karaya gum. Mixtures of said polymers may also be used to form substrates for use in the devices of the present invention. For example, PVA may be used in combination with one or more other suitable polymers (e.g. fenugreek gum, sesbania gum and/or cyclodextrin). The term "solvent-soluble substrate" refers to a substrate made from a material having a solubility in a given solvent of from about 0.1 to about 20 g per 100 ml solvent (such as from about 1 to about 20 g (e.g. about 10 g) per 100 ml solvent) under ambient conditions. The solvent-soluble substrate may be a water-soluble substrate. The solubility of the substrate in water may be from about 0.1 to about 20 g per 100 ml solvent (such as from about 1 to about 20 g (e.g. about 10 g) per 100 ml solvent) under ambient conditions.

Suitable polymers for use as solvent-soluble substrates include fenugreek gum, sesbania gum, cyclodextrin, PVA, and particularly gelatin, ammonium alginate, chitosan, copovidone, hydroxyethyl cellulose, hydroxypropyl cellulose, maltodextrin, polyethylene oxide, polyvinylpyrrolidone, polyvinylacetate, polyvinyl alcohol copolymers, polyvinylamine, polyacrylate salt and karaya gum. Mixtures of said polymers may also be used to form substrates. Solvent-soluble substrates are not according to the invention. In other preferred embodiments, the substrate is made from one or more polymers which polymers are cross-linked once they have been brought into contact with the drug delivery element. When the substrate is made from gelatin, cross-linking may be achieved using glutaraldehyde, or a similar cross-linking agent. Cross-linking may be performed for other substrate materials in order to strengthen the substrate once it has been brought into contact with the drug delivery element.

The transdermal drug administration device of the invention comprises a drug delivery element attached to a water-swellable substrate. The water-swellable substrate is generally brought into contact with the drug delivery element once that element has been formed. More particularly, the water-swellable substrate is preferably introduced once the ceramic or geopolymeric material has been cured or otherwise formed into a rigid solid.

The water-swellable substrate is typically introduced as a solution of substrate material in a solvent. This allows the substrate to be easily moulded to fit with the drug delivery element, and to become incorporated into the pores of the porous solid from which the drug delivery element is formed. The incorporation of the substrate into the pores of the porous solid greatly enhances the bonding of the substrate to the drug delivery element. In addition, this allows a strongly bonded device to be formed without the need to expose the porous solid, and any active ingredient contained within it, to harsh conditions which could have a deleterious effect on the active ingredient.

The transdermal drug administration devices of the invention provide for tunable, controlled and uniform release of active ingredient into a patient through the skin. This includes instant release as well as sustained or delayed release of the active ingredient.

Preferences and options for a given aspect, feature or parameter of the invention should, unless the context indicates otherwise, be regarded as having been disclosed in combination with any and all preferences and options for all other aspects, features and parameters of the invention.

For example, in a preferred embodiment, the invention relates to a transdermal drug administration device comprising a drug delivery element attached to a water-swellable substrate, wherein:
the drug delivery element defines a contact surface for location, in use, against a patient's skin, further wherein the drug delivery element comprises an active pharmaceutical ingredient and a porous solid material;
the water-swellable substrate is formed from one or more organic polymers selected from the group consisting of fenugreek gum, sesbania gum, cyclodextrin, PVA, and particularly silicon rubber, polymethyl methacrylate (PMMA), polydimethyl siloxane (PDMS), polyethylene (PE), polypropylene (PP), parylene, polyvinylpyrrolidone, polyvinylacetate, alginate (e.g. ammonium alginate), chitosan, gelatin, polyvinyl alcohol copolymers, polyacrylamide, carboxymethylcellulose, polyvinylimine, polyacrylate, karaya gum, copovidone, hydroxyethyl cellulose, hydroxypropyl cellulose, maltodextrin, polyethylene oxide, polyvinyl alcohol copolymers, polyvinylamine and polyacrylate salt; and
wherein the porous solid material is formed from a substance selected from the group consisting of calcium phosphates, calcium sulphates, calcium carbonates, calcium silicates, calcium aluminates, and magnesium carbonates (including solvates of any of the foregoing).

In a further preferred embodiment, the invention relates to a transdermal drug administration device comprising a drug delivery element attached to a water-swellable substrate, wherein:
the drug delivery element defines a contact surface for location, in use, against a patient's skin, further wherein the drug delivery element comprises an active pharmaceutical ingredient and a porous solid material;
the water-swellable substrate is a backing layer formed from gelatin; and
wherein the porous solid material is formed from a substance selected from the group consisting of a calcium phosphate, a calcium sulphate or a hydrate thereof.

As an alternative to gelatin in the above embodiment, the backing layer may be formed from PVA in combination with one or more polymers selected from the group consisting of fenugreek gum, sesbania gum and cyclodextrin.

The transdermal delivery device of the present invention is capable of containing one or more pharmaceutically active ingredients and allowing it or them to be delivered to a patient for the purposes of treating or preventing a disease or condition, or ameliorating the symptoms of a disease or condition. The transdermal drug administration devices of the present invention may be useful in delivering a wide range of drugs to a patient. The transdermal delivery device of the present invention is therefore useful in medicine.

The transdermal delivery device of the present invention is particularly useful for the delivery of drugs which require a relatively low dose and/or for drugs for which first-pass metabolism should be avoided. The transdermal delivery devices are also capable of providing sustained release of a particular active ingredient into the patient.

The active pharmaceutical ingredients employed in the drug delivery element preferably include substances from various pharmacological classes, e.g. antibacterial agents, antihistamines and decongestants, anti-inflammatory agents, antiparasitics, antivirals, local anaesthetics, antifungals, amoebicidals or trichomonocidal agents, analgesics, antianxiety agents, anticlotting agents, antiarthritics, antiasthmatics, anticoagulants, anticonvulsants, antidepressants, antidiabetics, antiglaucoma agents, antimalarials, antimicrobials, antineoplastics, antiobesity agents, antipsychotics, antihypertensives, auto-immune disorder agents, anti-impotence agents, anti-Parkinsonism agents, anti-Alzheimer's agents, antipyretics, anticholinergics, anti-ulcer agents, blood-glucose-lowering agents, bronchodilators, central nervous system agents, cardiovascular agents, cognitive enhancers, contraceptives, cholesterol-reducing agents, agents that act against dyslipidermia, cytostatics, diuretics, germicidials, hormonal agents, anti-hormonical agents, hypnotic agents, immunogenic agents, inotropics, muscle relaxants, muscle contractants, physic energizers, sedatives, sympathomimetics, vasodilators, vasocontrictors, tranquilizers, electrolyte supplements, vitamins, uricosurics, cardiac glycosides, membrane efflux inhibitors, membrane transport protein inhibitors, expectorants, purgatives, contrast materials, radiopharmaceuticals, imaging agents, peptides, enzymes, growth factors, vaccines, mineral trace elements. For the avoidance of doubt, the term "active pharmaceutical ingredients" includes peptides, proteins, antigens and immunogenic substances (e.g. vaccines) having appropriate pharmacological activity.

The active pharmaceutical ingredients preferably include any that are open to abuse potential, such as those that are useful in the treatment of acute or chronic pain, attention deficit hyperactivity disorders (ADHD), anxiety and sleep disorders, as well as growth hormones (e.g. erythropoietin), anabolic steroids, etc. A full list of potentially abusable substances may be found easily by the skilled person, for example see the active ingredients listed on the following weblink: http://www.deadiversion.usdoj.gov/schedules /alpha/alphabetical.htm.

Non-opioid drug substances that may be specifically mentioned include psychostimulants, such as modafinil, amphetamine, dextroamphetamine, methamphetamine and hydroxyamphethamine and, more preferably, methylfenidate; benzodiazepines, such as bromazepam, camazepam, chlordiazepoxide, clotiazepam, cloxazepam, delorazepam, estazolam, fludiazepam, flurazepam, halazepam, haloxazepam, ketazolam, lormetazepam, medazepam, nimetazepam, nordiazepam, oxazolam, pinazepam, prazepam, temazepam, tetrazepam and, more preferably, alprazolam, clonazepam, diazepam, flunitrazepam, lorazepam, midazolam, nitrazepam, oxazepam and triazolam; and other, non-benzodiazepine sedatives (e.g. short-acting hypnotics), such as zaleplon, zolpidem, zopiclone and eszopiclone.

Preferred pharmaceutically-active ingredients that may be employed in the composition include opioid analgesics. The term "opioid analgesic" will be understood by the skilled person to include any substance, whether naturally-occurring or synthetic, with opioid or morphine-like properties and/or which binds to opioid receptors, particularly the µ-opioid receptor, having at least partial agonist activity, thereby capable of producing an analgesic effect. The problems of potential formulation tampering and drug extraction by drug addicts are particularly prominent with opioids.

Opioid analgesics that may be mentioned include opium derivatives and the opiates, including the naturally-occurring phenanthrenes in opium (such as morphine, codeine, thebaine and Diels-Alder adducts thereof) and semisynthetic derivatives of the opium compounds (such as diamorphine, hydromorphone, oxymorphone, hydrocodone, oxycodone, etorphine, nicomorphine, hydrocodeine, dihydrocodeine, metopon, normorphine and N-(2-phenylethyl)normorphine). Other opioid analgesics that may be mentioned include fully synthetic compounds with opioid or morphine-like properties, including morphinan derivatives (such as racemorphan, levorphanol, dextromethorphan, levallorphan, cyclorphan, butorphanol and nalbufine); benzomorphan derivatives (such as cyclazocine, pentazocine and phenazocine); phenylpiperidines (such as pethidine (meperidine), fentanyl, alfentanil, sufentanil, remifentanil, ketobemidone, carfentanyl, anileridine, piminodine, ethoheptazine, alphaprodine, betaprodine, 1-methyl-4-phenyl-1,2,3,6-tetrahydropyridine (MPTP), diphenoxylate and loperamide), phenylheptamines or "open chain" compounds (such as methadone, isomethadone, propoxyphene and levomethadyl acetate hydrochloride (LAAM)); diphenylpropylamine derivatives (such as dextromoramide, piritramide, bezitramide and dextropropoxyphene); mixed agonists/antagonists (such as buprenorphine, nalorphine and oxilorphan) and other opioids (such as tilidine, tramadol and dezocine). Further opioid analgesics that may be mentioned include allylprodine, benzylmorphine, clonitazene, desomorphine, diampromide, dihydromorphine, dimenoxadol, dimepheptanol, dimethylthiambutene, dioxaphetyl butyrate, dipipanone, eptazocine, ethylmethylthiambutene, ethylmorphine, etonitazene, hydroxypethidine, levophenacylmorphan, lofentanil, meptazinol, metazocine, myrophine, narceine, norpipanone, papvretum, phenadoxone, phenomorphan, phenoperidine and propiram.

More preferred opioid analgesics include buprenorphine, alfentanil, sufentanil, remifentanil and, particularly, fentanyl.

Active pharmaceutical agents that are useful in treating diabetes that may be mentioned include insulin, metformin, glibenclamide, glipizide, gliquidone, glyclopyramide, glimepiride, gliclazide, repaglinide, nateglinide, alpha-glucosidase inhibitors (such as acarbose), rosiglitazone, pioglitazone, linagliptin, saxagliptin, sitagliptin, vildagliptin, dulaglutide, exenatide, liraglutide, lixisenatide, amylin and pramlintide.

Other preferred active pharmaceutical ingredients include benzodiazepines, clonidine and zolpidem, and pharmaceutically acceptable salts thereof.

Additional active pharmaceutical ingredients that may be mentioned in the context of the present invention include antigens (which may form the basis of a vaccine) and/or enzymes.

The transdermal drug administration devices of the present invention may be useful in delivering a wide range of active pharmaceutical ingredients to a patient. In addition to the drugs disclosed above, the transdermal drug administration devices of the present invention may be useful in delivering vaccines to patients. For example, the transdermal drug administration devices may be useful in delivering vaccines for diseases such as influenza and ebola.

Particularly preferred active pharmaceutical ingredients include antihypertensives, sedatives, hypnotics, analgesics and immunogenic substances (e.g. vaccines).

Active ingredients listed above may also be formulated in the composition in any specific combination.

In the case of drug administration devices comprising opioid analgesics, in order to further improve abuse-deterrent properties, an opioid antagonist with limited or no transdermal absorption may be included in the composition together with the opioid. Any attempt to tamper with the formulation for subsequent injection will also release the antagonist and therefore potentially prevent the desired abuse-generated pharmacological effect. Examples of opioid antagonists and partial opioid antagonists include naloxone, naltrexone, nalorphine and cyclazocine.

Active pharmaceutical ingredients may further be employed in salt form or any other suitable form, such as e.g. a complex, solvate or prodrug thereof, or in any physical form such as, e.g., in an amorphous state, as crystalline or part-crystalline material, as co-crystals, or in a polymorphous form or, if relevant, in any stereoisomeric form including any enantiomeric, diastereomeric or racemic form, or a combination of any of the above.

Pharmaceutically-acceptable salts of active ingredients that may be mentioned include acid addition salts and base addition salts. Such salts may be formed by conventional means, for example by reaction of a free acid or a free base form of an active ingredient with one or more equivalents of an appropriate acid or base, optionally in a solvent, or in a medium in which the salt is insoluble, followed by removal of said solvent, or said medium, using standard techniques (e.g. *in vacuo,* by freeze-drying or by filtration). Salts may also be prepared by exchanging a counter-ion of active ingredient in the form of a salt with another counter-ion, for example using a suitable ion exchange resin.

Examples of pharmaceutically acceptable addition salts include those derived from mineral acids, such as hydrochloric, hydrobromic, phosphoric, metaphosphoric, nitric and sulphuric acids; from organic acids, such as tartaric, acetic, citric, malic, lactic, fumaric, benzoic, glycolic, gluconic, succinic, arylsulphonic acids; and from metals such as sodium, magnesium, or preferably, potassium and calcium.

The transdermal drug administration devices of the present invention may be manufactured according to the methods described herein. In one aspect of the invention, there is disclosed a method of manufacturing the transdermal drug administration device of the invention, which method comprises the steps of:
(a) preparing the drug delivery element as defined herein;
(b) incorporating the active pharmaceutical ingredient as defined herein into or onto the drug delivery element; and
(c) coating a surface of the drug delivery element with a water-swellable substrate comprising one or more organic polymers as defined herein.

According to the invention, the drug delivery element is formed from one or more ceramic materials or one or more geopolymer materials. The throne or more ceramic materials or one or more geopolymer materials may be formed in a mould and allowed to set (or be cured in some way) to form a solid structure. Such a solid structure may have a contact surface for location, in use, against a patient's skin, and a second surface for contact with the water-swellable substrate. Once the material comprising the drug delivery element is set (or cured), a surface of the drug delivery element may then be coated with the water-swellable substrate.

In such methods, the active pharmaceutical ingredient may be incorporated into the device at any stage during the manufacturing method. For example, it may be incorporated into the drug delivery element prior to, or during, the formation of the drug delivery element (e.g. prior to the curing of the precursor material). Alternatively, the active pharmaceutical ingredient may be incorporated into, or coated onto, the drug delivery element after it is formed, in which case it is preferable (but not essential) that the active pharmaceutical ingredient is incorporated into, or coated onto, the drug delivery element after the water-swellable substrate has been coated onto a surface of the drug delivery element. In embodiments in which the active pharmaceutical ingredient is coated onto the drug delivery element, that active ingredient may be combined with the drug delivery element by means of spraying, brushing, rolling, dip coating, powder coating, misting and/or chemical vapour deposition.

In a particularly preferred embodiment of this aspect of the invention:
(a) the active pharmaceutical ingredient is mixed with the ingredients required to prepare the drug delivery element;
(b) the drug delivery element is formed from the mixture obtained in (a); and
(c) the drug delivery element formed in (b) is coated with a water-swellable substrate.

In another particularly preferred embodiment of this aspect of the invention:
(a) the drug delivery element is formed;
(b) the active pharmaceutical ingredient brought into association with the drug delivery element by coating the element with, and/or soaking the element in, the active pharmaceutical ingredient (or a solution containing said active ingredient); and
(c) a water-swellable substrate is applied to the product of step (b).

The drug delivery element of the transdermal drug administration device contains a pharmacologically effective amount of the active ingredient. By "pharmacologically effective amount", we refer to an amount of active ingredient, which is capable of conferring a desired therapeutic effect on a treated patient (which may be a human or animal (e.g. mammalian) patient), whether administered alone or in combination with another active ingredient. Such an effect may be objective (i.e. measurable by some test or marker) or subjective (i.e. the subject gives an indication of, or feels, an effect).

Preferably the drug delivery element may be adapted (for example as described herein) to provide a sufficient dose of drug over the dosing interval to produce a desired therapeutic effect.

The amounts of active ingredients that may be employed in the drug delivery element may thus be determined by the physician, or the skilled person, in relation to what will be most suitable for an individual patient. This is likely to vary with the type and severity of the condition that is to be treated, as well as the age, weight, sex, renal function, hepatic function and response of the particular patient to be treated. A particular advantage of the drug administration devices of the present invention is that they may be suitable for drug administration to children. The devices may also be used (with adults and, particularly, children), for premedication prior to surgery.

Drug delivery elements comprising antihypertensive agents such as clonidine and its salts (particularly clonidine hydrochloride) are useful in the treatment of hypertension (high blood pressure). A further aspect of the invention is the transdermal drug administration device of the invention for use in a method of treatment of hypertension which comprises locating a contact surface of such a drug delivery element of a transdermal drug administration device according to the invention against the skin of a patient suffering from, or susceptible to, such a condition. In another embodiment, there is provided the use of the transdermal drug administration device of the present invention for the manufacture of a medicament for the treatment of hypertension.

Clonidine hydrochloride is also useful in the treatment of additional conditions such as anxiety disorders, hyperactivity disorder and pain. Therefore, a further aspect of the invention is the transdermal drug administration device of the invention for use in a method of treatment of anxiety disorders, hyperactivity disorder or pain which comprises locating a contact surface of such a drug delivery element of a transdermal drug administration device according to the invention against the skin of a patient suffering from, or susceptible to, such a condition. In another embodiment, there is provided the use of the transdermal drug administration device of the present invention for the manufacture of a medicament for the treatment of anxiety disorders, hyperactivity disorder or pain.

When the drug delivery element comprises one or more opioid analgesics, appropriate pharmacologically effective amounts of such opioid analgesic compounds include those that are capable of producing (e.g. sustained) relief of pain when administered. References to pain herein include references to post-operative pain.

Drug delivery elements comprising opioid analgesics are useful in the treatment of pain, particularly severe and/or chronic pain. A still further aspect of the invention is the transdermal drug administration device of the invention for use in a method of treatment of pain which comprises locating a contact surface of such a drug delivery element of a transdermal drug administration device according to the invention against the skin of a patient suffering from, or susceptible to, such a condition. In another embodiment, there is provided the use of the transdermal drug administration device of the present invention for the manufacture of a medicament for the treatment of pain.

When the drug delivery element comprises one or more hypnotics, appropriate pharmacologically effective amounts of such compounds include those that are capable of producing (e.g. sustained) relief from insomnia when administered.

Drug delivery elements comprising hypnotics, such as a benzodiazepine or zolpidem, are useful in the treatment of insomnia. A still further aspect of the invention is the transdermal drug administration device of the invention for use in a method of treatment of insomnia which comprises locating a contact surface of such a drug delivery element of a transdermal drug administration device according to the invention against the skin of a patient suffering from, or susceptible to, such a condition. In another embodiment, there is provided the use of the transdermal drug administration device of the present invention for the manufacture of a medicament for the treatment of insomnia.

Drug delivery elements comprising drugs useful in treating diabetes, such as metformin or insulin, are useful in the treatment of diabetes. A still further aspect of the invention is the transdermal drug administration device of the invention for use in a method of treatment of diabetes which comprises locating a contact surface of such a drug delivery element of a transdermal drug administration device according to the invention against the skin of a patient suffering from, or susceptible to, such a condition. In another embodiment, there is provided the use of the transdermal drug administration device of the present invention for the manufacture of a medicament for the treatment of diabetes.

For the avoidance of doubt, by "treatment" we include the therapeutic (including curative) treatment, as well as the symptomatic treatment, the prophylaxis, or the diagnosis, of the condition.

Transdermal drug administration devices of the invention possess the advantage that the swelling of the water-swellable substrate in use (i.e. when the transdermal drug administration device is brought into contact with the skin of the patient) facilitates the penetration of the drug delivery element into the outer layers of the skin. Without wishing to be bound by theory, it is believed that the increase in the volume of the substrate leads to forces being transferred to the drug delivery element in the direction of the skin. When the drug delivery element comprises microscopic protrusions, those microscopic protrusions are able to penetrate the skin of the patient, and the swelling of the substrate leads to penetration which is more enhanced and consistent across the entire drug delivery element.

In addition, the water-swellable substrate may act as a supporting layer and may simultaneously assist with the drug delivery in other ways. The binding between the drug delivery element and the substrate used in the present invention may be particularly strong immediately following manufacture of the drug administration device. After contact with a bodily fluid, the substrate can swell and separate from the drug delivery element, leaving the element in contact with the patient's skin. In embodiments in which the delivery element comprises an array of microscopic protrusions, the separation of the substrate from the drug delivery element may advantageously leave the microscopic protrusions embedded within the patient's skin allowing the protrusions to act as a drug depot. This also reduces the risk of accidental microneedle removal and any discomfort and inconvenience caused by the substrate during the drug delivery.

Transdermal drug administration devices of the invention may also have the advantage that the manufacturing process does not require harsh conditions (e.g. high temperatures) which could be detrimental to the performance of the active ingredient(s) comprised within the device.

Transdermal drug administration devices of the invention also possess the advantage of the avoidance and/or reduction of the risk of either dose dumping (i.e. the involuntary release), or equally importantly the deliberate *ex vivo* extraction, of the majority (e.g. greater than about 50%, such as about 60%, for example about 70% and in particular about 80%) of the dose of the active ingredient(s) that is initially within the composition included in the drug delivery element, within a timeframe that is likely to give rise to undesirable consequences, such as adverse pharmacological effects, or the potential for abuse of that active ingredient (for example when such release is deliberately effected *ex vivo* by an individual).

Transdermal drug administration devices of the invention may also have the advantage that the composition included in the drug delivery element may be prepared using established pharmaceutical processing methods and may employ materials that are approved for use in foods or pharmaceuticals or of like regulatory status.

Transdermal drug administration devices of the invention may also have the advantage that the composition included in the drug delivery element may be more efficacious than, be less toxic than, be longer acting than, be more potent than, produce fewer side effects than, be more easily absorbed than, and/or have a better pharmacokinetic profile than, and/or have other useful pharmacological, physical, or chemical properties over, pharmaceutical compositions known in the prior art, whether for use in the treatment of pain or otherwise.

Wherever the word "about" is employed herein in the context of dimensions (e.g. values, temperatures, pressures (exerted forces), relative humidities, sizes and weights, particle or grain sizes, pore sizes, timeframes etc.), amounts (e.g. relative amounts (e.g. numbers or percentages) of particles, individual constituents in a composition or a component of a composition and absolute amounts, such as doses of active ingredients, numbers of particles, etc), deviations (from constants, degrees of degradation, etc) it will be appreciated that such variables are approximate and as such may vary by ± 10%, for example ± 5% and preferably ± 2% (e.g. ± 1%) from the numbers specified herein.

The invention is illustrated by the following examples in which:
Figure 1 shows the micro-molding process for fabricating the ceramic microneedles with the flexible backing layer.
Figure 2 shows SEM, fluorescent 3-D images of BCMN-G: Cross-section view of BCMN-G450 (a); magnification of BCMN-G450 (b); 3-D reconstruction image of BCMN-G450 (c); cross-section view of BCMN-G600 (d); magnification of BCMN-G600 (e); 3-D reconstruction image of BCMN-G600 (f); magnification of microneedle tips (g); magnification of the pyramid's foot (h); magnification of interface between needle and substrate (i).
Figure 3 shows a custom-build vertical diffusion cell.
Figure 4 shows drug release from the BCMN-G450 and BCMN-G600: average of drug release fraction from preloaded with clonidine a); average of drug release fraction from coated with clonidine b); drug release fraction from the 10 repeats of preloaded BCMN-G450 c); drug release fraction from the 10 repeats of coated BCMN-G450 d).
Figure 5 shows SEM image of: the BCMN-G600 before drug release (a); BCMN-G600 after drug release (b); membrane before drug release (c); and membrane after drug release (d).
Figure 6 shows: light microscopy images of porcine skin after manual insertion of BCMN-G450 a); light microscopy image of porcine skin after manual insertion of BCMN-G600 b); magnified image of porcine skin showing the insertion mark and broken stratum corneum c) and d).
Figure 7a shows a synthetic skin simulator used to compare drug release from the SCMNs.
Figure 7b shows a vertical diffusion cell used to compare the drug release from the BCMN-Gs.
Figure 8 shows the drug release profile for the BCMN-Gs.
Figure 9 shows the drug release profile for the SCMNs.

### Example 1 - Fabrication of bioceramic microneedles with flexible and self-swelling substrate (BCMN-G)

To manufacture the needle patches, master templates were first prepared by microfabrication methods on stainless steel. Two templates used for this study were: 450µm in height, 285µm in base width and 820µm between tips and 600µm in height, 380µm in base width and 916µm between tips. For both design the needle tip radius was 5µm. These lengths were selected in order to reach the viable epidermis after piercing the stratum corneum. A negative replica of the master template, made of commercial available synthetic silicone, was prepared as intermediate.

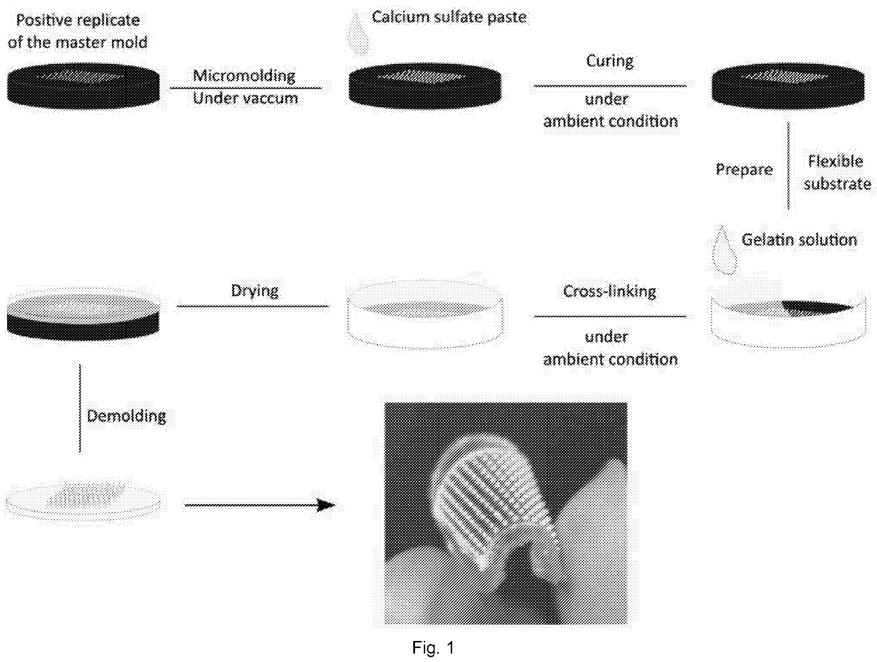

Alpha calcium sulfate hemihydrate (particle size <100µm) was well mixed with model drug and water into a homogenous paste (powder/liquid ratio as 2.5). To prepare the bioceramic microneedles, ceramic paste was filled into the cavities in the positive replicas. The needles were cured in ambient condition for at least 10 hours. Warmed gelatin solution (0.2g/ml) was then poured on the top of mould and cross-linked in the desiccators with 2% of aqueous glutaraldehyde solution overnight under ambient condition. The micro-molding process for fabricating the ceramic microneedle with flexible backing layer is illustrated in Figure 1.

### Example 2 - Characterization of BCMN-G

The finished microneedle arrays, BCMN-G450 (for microneedles having a height of 450µm) and BCMN-G600 (for microneedles having a height of 600µm), were observed under scanning electron microscope (SEM) using Leo 1550 FEG microscope (Zeiss, UK). To get a better observation of the microneedle, rhodamine B was blended into the ceramic paste and the resultant needles were observed under Eclipse TE2000-E inverted microscope (Nikon, Melville, NY).

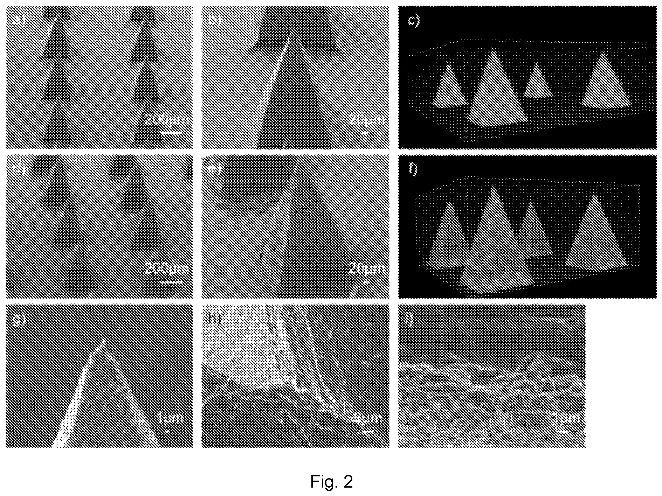

The bioceramics were developed into the pyramid-shape needles with sharp tips (radius can be less than 5 µm) (Figure 2a-b and d-e). The surface of the ceramic needles was rough with abundant pores and channels (Figure 2g). Therefore, it was concluded that the gelatin substrate could penetrate into the pores and channels in the needle base and form a tight micromechanical binding with the needle arrays (Figure 2h and i). BCMN-G loaded with rodamine B was imaged using confocal fluorescent microscope. The 3-D reconstruction from the fluorescent images indicated that the loaded fluorescent dye could homogenously distribute in the needles from tip to base (Figure 2c and f).

### Example 3 - Drug delivery studies

Clonidine hydrochloride was used as the model drug in this study. The drug was loaded into BCMN-Gs by direct mixing or coating. For the direct mixing BCMN-Gs, the drug powder was homogenously mixed into ceramic paste before fitting into negative mould. To prepare the drug coating for the other BCMN-Gs, 50µl clonidine ethanol solution (20mg/ml) was spread on the array surface and dried at room conditions.

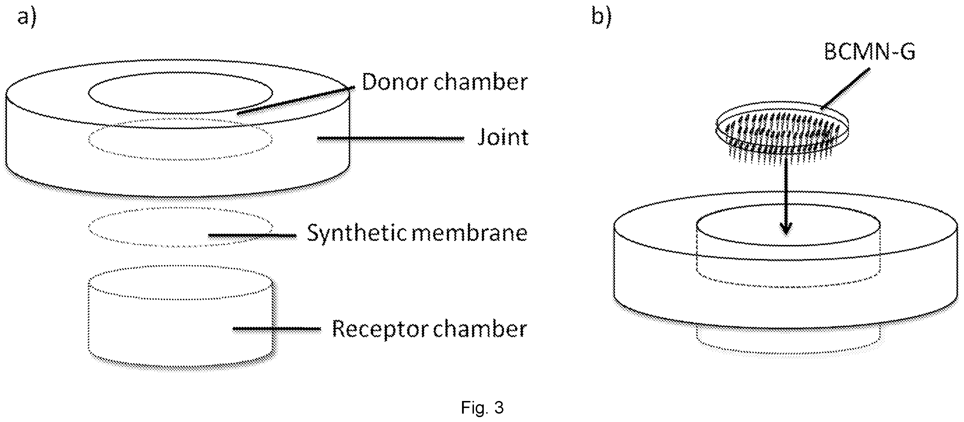

The drug release from BCMN-G was investigated in vitro using a custom-build vertical diffusion cell (Figure 3). Synthetic membrane (47mm, 0.4µm, Nuclepore®, Whatman) was first equilibrated with water and fixed between donor and receiver chamber. BCMN-G loaded with clonidine hydrochloride was placed on the synthetic membrane. The receptor chamber was filled with 20ml distillated water and the donor chamber was sealed using Parafilm® (Alpha Laboratories, Hampshire, UK) to reduce the evaporative loss. The diffusion cell was situated at 37°C on a shaker to maintain temperature of skin and good mixing. At the predetermined time intervals (0.5, 1, 2 and 4 hours), 1ml aliquots were withdrawn from the diffusion cell by a syringe and replaced by 1ml distillated water. The concentrations of clonidine hydrochloride in the solutions were analysed using isocratic reversed-phase high-performance liquid chromatography (HPLC) with a photodiode array detector (Waters, Corp., Milford, MA, USA) and a YMC-Triart C18 column (2.0 mm ID × 12 mm, 3 µm; YMC, Japan). The fraction of drug released was calculated from the total drug content in the formulation. The remaining needle array and membrane were collected and observed under scanning electron microscope (SEM, Leo 1550 FEG, Zeiss, UK).

The drug release from BCMN-G450 and BCMN-G600, loaded with drug using two different methods as mentioned above, was evaluated in a vertical diffusion cell using a synthetic membrane. This membrane was chosen given that it had low diffusional resistance and enough mechanical strength to separate two chambers. BCMN-Gs were compared to controls comprising of drug-loaded needles with solid backing layer.

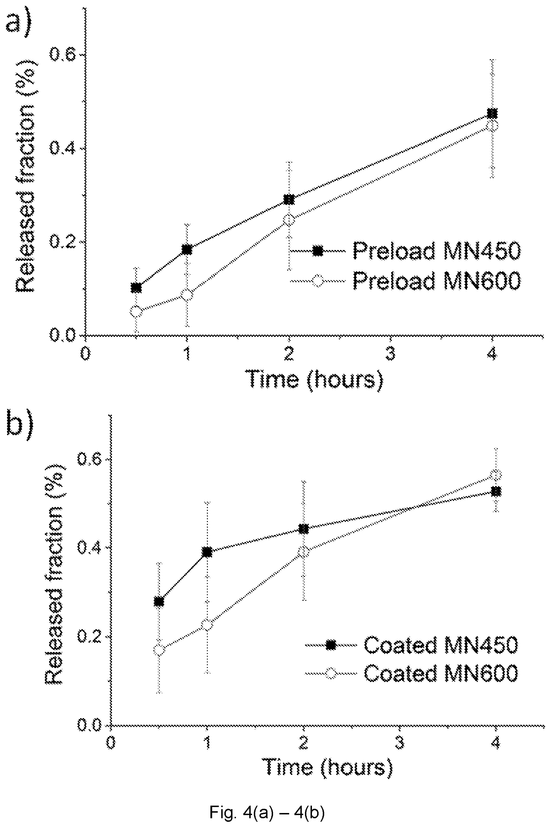

Clonidine hydrochloride was used as model drug in this study. All BCMN-G released the drug in a sustained manner: 45%-55% of the drug content was released within 4 hours (Figure 4a and b). The microneedle geometry, i.e. BCMN-G450 and BCMN-G600, did not influence much on the drug release i.e. p>0.05 evaluated by t-test. The needles coated with the drug released significantly faster than the needles with the drug incorporated in the arrays (p≤0.05). The results also show that clonidine release from the needles was at an approximately constant rate during the first four hours.

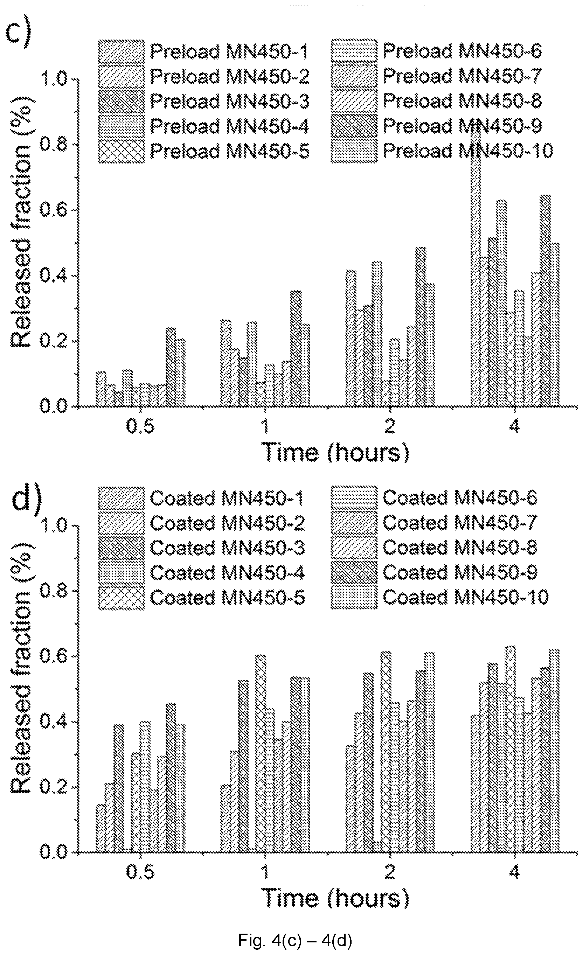

Figure 4c and d show the variation between the drug-release fraction from each preloaded and coated BCMN-G450. The variance between samples was thought mainly due to the uneven water absorption of gelatin substrate. The gelatin layer was covered on the back of the needles, cross-linked and dried in the air. The process made it hard to control the substrate as a flat, uniform surface. The irregular surface of the substrate would cause the differences in water sorption and thus drug release.

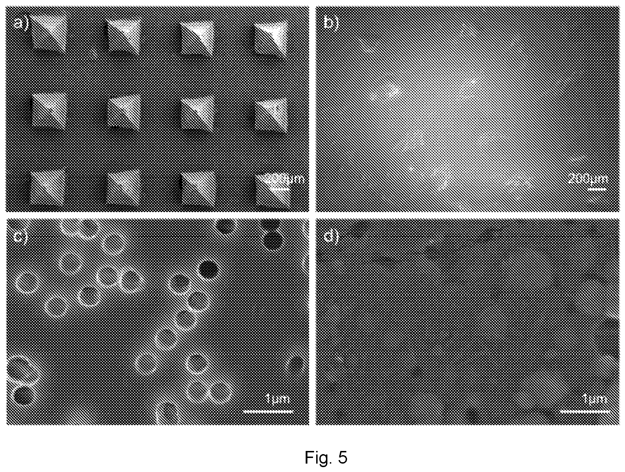

BCMN-G and synthetic membrane before and after drug release were observed using SEM (Figure 5). After four-hour drug release, no obvious needle residue was found on the gelatin substrate. The marks left on the substrate generally matched the distance between the needles (Figure 5a and b). The position of the mark did not match exactly, probably due to the swelling and drying of the substrate during drug release. After the drug release, the membrane was covered with a layer of gelatin and calcium sulfate crystals, which conceal the pore features on the membrane (Figure 5c and d). The crystal residue remained on the membrane indicating that the needles were degraded after contact with the water and recrystallized. Therefore, we believe that the mechanism of the drug release depend both on the diffusion of drug molecules through the ceramic pores and the degradation of the bioceramic needles.

### Example 4 - Skin penetration studies

Full thickness of porcine ear skin was chosen to evaluate the penetration ability of BCMN-G. The pig ear skin is an excellent model showing similar skin layer thickness as human skin. The capability of insertion of BCMN-G was investigated using the full-thickness porcine skin.

Freshly excised porcine ears, from pigs, were obtained from a local abattoir and washed under cold running water. Full thickness skin was isolated from the underlying cartilage by blunt dissection and any hairs removed using clippers. The skin was cut into sections (∼3cm²) and stored at -70°C until required.

Sections of frozen skin were thawed by soaking in water and then dabbed with absorbent paper to remove excess moisture on the surface. BCMN-G was applied to the skin section with gentle thumb pressure for 30 seconds. The needles were removed instantly after the insertion and the skin was sectioned and embedded in OCT compound in a cryostat mould. The OCT-skin samples were frozen and sliced into sections with 30µm in thickness. The histological sections were placed on glass slides and observed using Eclipse TE2000-E (Nikon, Melville, NY).

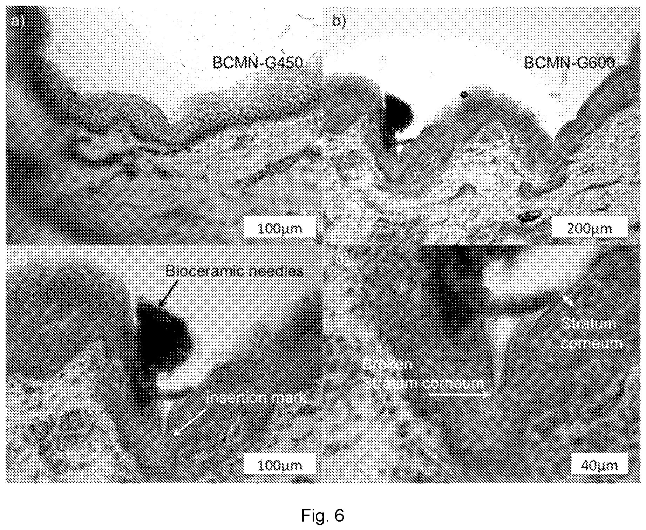

The histology cross-section of the skin showed that a sharp mark had been imprinted on skin and the stratum corneum were broken after manual insertion of BCMN-G600 (Figure 6). As expected, the insertion mark caused by BCMN-G600 was deeper than that by BCMN-G450. Moreover, some intact tips of bioceramic needles were found embedded in skin.

### Example 5 - Drug release studies

### Drug delivery elements used in this test

### (i) Structure

| | **Base width** | **Height** | **Tip to tip distance** | **Array size** |
|---|---|---|---|---|
| SCMN-sparsely arranged | 250 | 200 | 820 | |
| SCMN-densely arranged | 150 | 100 | 150 | |
| BCMN-G600 | 380 | 600 | 916 | 13 x 13 |
| BCMN-G450 | 285 | 450 | 820 | 15 x 15 |

### (ii) Composition

The ceramic compositions of SCMNs and BCMN-Gs are the same.

| | Calcium sulfate hemihydrate | Water |
|---|---|---|
| Amount | 5g | 2mL |

Drug loading: SCMNs contains 1.14wt% or 2.25wt% drug in ceramics. Each SCMN array contains 15 or 30 mg of zolpidem. BCMN-G contains 10.7wt% drug in ceramics. Each BCMN-G array contains 10-15mg of clonidine hydrochloride.

Drug was loaded in the needles and solid backing as well for SCMNs. But drug was only loaded in the needles for BCMN-Gs.

### Testing method

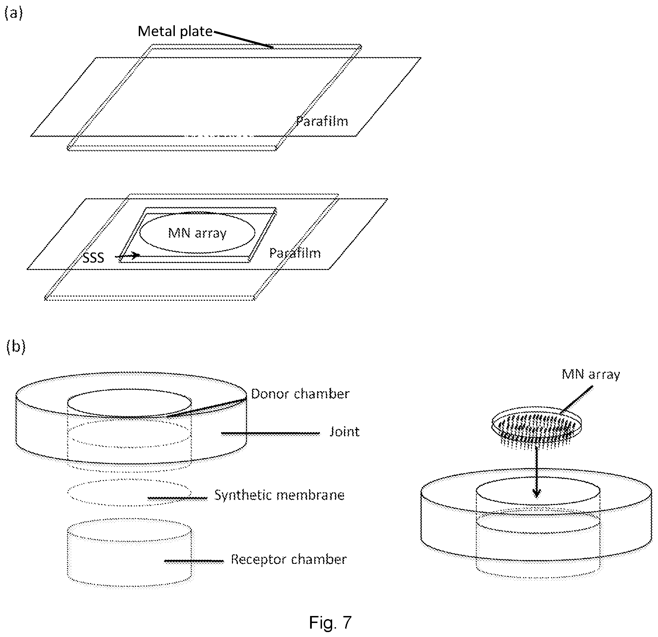

A bench-top method based on synthetic skin simulator (SSS) was used to compare the drug release from different SCMNs (see Figure 7a). As the amount of moisture accessible to the microneedle in the skin was limited, the *in vitro* dissolution tests, USP II, are not suitable to evaluate the performance. The SSS method, which was validated using a commercial transdermal patch, is an easy-to-handle test method and suitable to provide preliminary screening of transdermal and geopolymer-based formulations under limited humidity. A piece of cellulose drug reservoir (Wettex®, Freudenberg, Sweden) was prepared in squared shape (2 x 2 cm²) and moistened with 400µl of pH 6.8±0.5 phosphate buffer. An SCMN plate was placed on the cellulose reservoir, i.e. synthetic skin simulator (SSS), and covered with Parafilm® to reduce the vaporization. The released drug was collected on the SSS during the testing. At predetermined time points, SSS was collected and replaced by a new piece of moisturized SSS. The drug containing SSS was then soaked in pH 1±0.5 HCI aqueous solution to release the collected drug molecules. The drug concentration was measured by a UV/VIS spectrophotometer.

A vertical diffusion cell was used to compare the drug release from the BCMN-G (Figure 7b). The diffusion cell was composed of two chambers: receptor and donor. The receptor chamber was filled with 20mL distilled water. A synthetic membrane was placed in between the chambers and fixed in position by the joint. Air was minimized under the membrane when positioning the membrane and joint. The microneedle array was placed on the membrane in the donor chamber and covered with Parafilm® to avoid any evaporation. The diffusion cell was placed in 37°C on a shaker during drug release. Aliquots of 1mL were collected from the receptor chamber and replaced by 1mL of distilled water. The drug concentration was determined using HPLC as described above.

### Performance

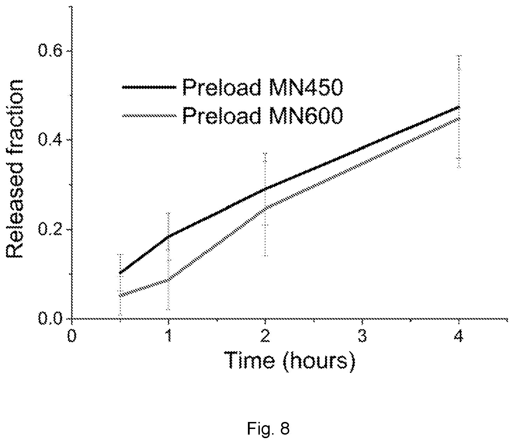

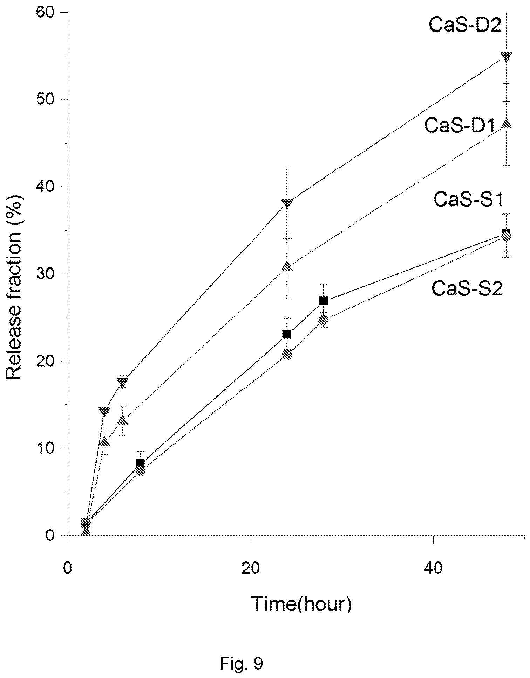

BCMN-Gs released around 50% of drug content during first 4 hours (Figure 8) while SCMNs released around 10% of the drug content (Figure 9). The differences are mainly due to the design of the microneedle array. Firstly, as the drug was only loaded in the needle part of BCMN-G array, the total drug content was much less than in the SCMNs. As a result, it could reduce the waste of unreleased dose in the microneedles and reduce the diffusion distance during delivery. In addition, the swellable substrate would propel the needle, which increases the surface area that could be exposed to facilitate further release and disintegration of the needles.

### Example 6 - Microneedles with rapidly dissolvable backing layers (not according to the invention)

Microneedle patches were prepared using the following mixtures for the backing layer:
Ex. 6a: PVA (polyvinyl alcohol) and fenugreek gum
Ex. 6b: PVA and Sesbania gum
Ex. 6c: PVA and Cyclodextrin

An aqueous solution of the relevant ingredients (e.g. PVA and fenugreek gum for the patch of Example 6a) was prepared with a range of weight ratios. The solutions were placed on a ceramic layer containing the microneedles. The backing layer was air-dried at room temperature for between 2 and 24 hours.

In each microneedle, the amount of PVA was greater than or equal to 60wt% relative to the weight of the other components in the backing layer. The composition and thickness of the backing layer affect the dissolution rate.

Present testing was performed on the wet cloth. The backing layer could be peeled away from the microneedles within as little as 2 minutes. The backing layer could be also dissolved within as little as 5 minutes.

## Claims

1. A transdermal drug administration device comprising a drug delivery element attached to a water-swellable substrate comprising one or more organic polymers, wherein the drug delivery element defines a contact surface for location, in use, against a patient's skin, further wherein the drug delivery element comprises an active pharmaceutical ingredient and a porous solid material, wherein the porous solid material is based on one or more ceramic materials or one or more geopolymeric materials.

2. A transdermal drug administration device according to Claim 1 wherein the porous solid material is formed from a self-setting ceramic.

3. A transdermal drug administration device according to Claim 1 or Claim 2, wherein the porous solid material is based on a calcium sulfate, a calcium phosphate, a calcium silicate, a calcium carbonate or a magnesium carbonate; optionally wherein the porous solid material is calcium sulfate hemihydrate or acidic calcium phosphate.

4. A transdermal drug administration device according to any one of the preceding claims, wherein:
(a) the active pharmaceutical ingredient is predominantly located within the pores of the porous solid; or
(b) the active pharmaceutical ingredient is predominantly located on the outer surface of the porous solid.

5. A transdermal drug administration device according to any one of the preceding claims, wherein the drug delivery element is an array of microscopic protrusions.

6. A transdermal drug administration device according to any one of the preceding claims, wherein the contact surface of the drug delivery element is moulded to define an array of microscopic protrusions; optionally wherein:
(a) the contact surface of the drug delivery element is moulded to define an array of microscopic conical or pyramidal protrusions; or
(b) the contact surface of the drug delivery element is moulded to define an array of micro-needles.

7. A transdermal drug administration device according to any one of the preceding claims wherein the water-swellable substrate is deformable against a patient's skin.

8. A transdermal drug administration device according to any one of Claims 1 to 7, wherein the one or more organic polymers is selected from the group consisting of fenugreek gum, sesbania gum, cyclodextrin, PVA, silicon rubber, polymethyl methacrylate, polydimethyl siloxane, polyethylene, polypropylene, parylene, polyvinylpyrrolidone, polyvinylacetate, alginate (e.g. ammonium alginate), chitosan, gelatin, polyvinyl alcohol copolymers, polyacrylamide, carboxymethylcellulose, polyvinylimine, polyacrylate, karaya gum, copovidone, hydroxyethyl cellulose, hydroxypropyl cellulose, maltodextrin, polyethylene oxide, polyvinylamine and polyacrylate salt.

9. A transdermal drug administration device according to any preceding claim wherein the active pharmaceutical ingredient is an antihypertensive, a sedative, a hypnotic, an analgesic or an immunogenic substance.

10. A transdermal drug administration device according to Claim 9 wherein the active pharmaceutical ingredient is selected from a benzodiazepine, clonidine and zolpidem, or a pharmaceutically acceptable salt thereof.

11. A transdermal drug administration device according to any one of Claims 1 to 9, wherein the active pharmaceutical ingredient is a peptide or a protein.

12. A transdermal drug administration device according to any one of the preceding claims for use in therapy.

13. A transdermal drug administration device according to Claim 9 or Claim 10 for use in the treatment of insomnia, hypertension, anxiety disorders, hyperactivity disorder, pain or diabetes.

14. A transdermal drug administration device according to any one of Claims 1 to 9 for use in the treatment or prevention of pain, wherein the active pharmaceutical ingredient is an opioid analgesic and the device is used to premedicate a patient prior to surgery.

15. A method of manufacturing a transdermal drug administration device as defined in any one of Claims 1 to 11, which method comprises the steps of:
(a) preparing a drug delivery element as defined in any one of Claims 1 to 6;
(b) incorporating the active pharmaceutical ingredient into or onto the drug delivery element; and
(c) coating a surface of the drug delivery element with a water-swellable substrate comprising one or more organic polymers.

## Patentansprüche

1. Transdermale Arzneimittelverabreichungsvorrichtung, die ein Arzneimittelzuführungselement umfasst, das an einem wasserquellbaren Substrat befestigt ist, das ein oder mehrere organische Polymere umfasst, wobei das Arzneimittelzuführungselement eine Kontaktoberfläche zum Platzieren, in Verwendung, an der Haut eines Patienten definiert, ferner wobei das Arzneimittelzuführungselement einen pharmazeutischen Wirkstoff und ein poröses Vollmaterial umfasst, wobei das poröse Vollmaterial auf einem oder mehreren keramischen Materialien oder auf einem oder mehreren geopolymeren Materialien basiert.

2. Transdermale Arzneimittelverabreichungsvorrichtung nach Anspruch 1, wobei das poröse Vollmaterial aus einem selbsthärtenden keramischen Werkstoff ausgebildet ist.

3. Transdermale Arzneimittelverabreichungsvorrichtung nach Anspruch 1 oder 2, wobei das poröse Vollmaterial auf einem Calciumsulfat, einem Calciumphosphat, einem Calciumsilicat, einem Calciumcarbonat oder einem Magnesiumcarbonat basiert; optional wobei das poröse Vollmaterial Calciumsulfathalbhydrat oder saures Calciumphosphat ist.

4. Transdermale Arzneimittelverabreichungsvorrichtung nach einem der vorhergehenden Ansprüche, wobei:
(a) der pharmazeutische Wirkstoff vorwiegend innerhalb der Poren des porösen Feststoffs positioniert ist; oder
(b) der pharmazeutische Wirkstoff vorwiegend auf der äußeren Oberfläche des porösen Feststoffs positioniert ist.

5. Transdermale Arzneimittelverabreichungsvorrichtung nach einem der vorhergehenden Ansprüche, wobei das Arzneimittelzuführungselement eine Anordnung von mikroskopischen Überständen ist.

6. Transdermale Arzneimittelverabreichungsvorrichtung nach einem der vorhergehenden Ansprüche, wobei die Kontaktoberfläche des Arzneimittelzuführungselements geformt ist, um eine Anordnung von mikroskopischen Überständen zu definieren; optional wobei:
(a) die Kontaktoberfläche des Arzneimittelzuführungselements geformt ist, um eine Anordnung von mikroskopischen kegelförmigen oder pyramidenförmigen Überständen zu definieren; oder
(b) die Kontaktoberfläche des Arzneimittelzuführungselements geformt ist, um eine Anordnung von Mikronadeln zu definieren.

7. Transdermale Arzneimittelverabreichungsvorrichtung nach einem der vorhergehenden Ansprüche, wobei das wasserquellbare Substrat an der Haut eines Patienten verformbar ist.

8. Transdermale Arzneimittelverabreichungsvorrichtung nach einem der Ansprüche 1 bis 7, wobei das eine oder die mehreren organischen Polymere aus der Gruppe ausgewählt sind, die aus den Folgenden besteht: Bockshornkleegummi, Sesbania-Gummi, Cyclodextrin, PVA, Siliciumkautschuk, Polymethylmethacrylat, Polydimethylsiloxan, Polyethylen, Polypropylen, Parylen, Polyvinylpyrrolidon, Polyvinylacetat, Alginat (z. B. Ammoniumalginat), Chitosan, Gelatine, Polyvinylalkoholcopolymere, Polyacrylamid, Carboxymethylcellulose, Polyvinylimin, Polyacrylat, Karayagummi, Copovidon, Hydroxyethylcellulose, Hydroxypropylcellulose, Maltodextrin, Polyethylenoxid, Polyvinylamin und Polyacrylatsalz.

9. Transdermale Arzneimittelverabreichungsvorrichtung nach einem der vorhergehenden Ansprüche, wobei der pharmazeutische Wirkstoff eine antihypertensive, eine sedierende, eine hypnotische, eine analgetische oder eine immunogene Substanz ist.

10. Transdermale Arzneimittelverabreichungsvorrichtung nach Anspruch 9, wobei der pharmazeutische Wirkstoff aus einem Benzodiazepin, Clonidin und Zolpidem oder einem pharmazeutisch unbedenklichen Salz davon ausgewählt ist.

11. Transdermale Arzneimittelverabreichungsvorrichtung nach einem der Ansprüche 1 bis 9, wobei der pharmazeutische Wirkstoff ein Peptid oder ein Protein ist.

12. Transdermale Arzneimittelverabreichungsvorrichtung nach einem der vorhergehenden Ansprüche zur Verwendung bei einer Therapie.

13. Transdermale Arzneimittelverabreichungsvorrichtung nach Anspruch 9 oder 10 zur Verwendung beim Behandeln von Insomnie, Hypertonie, Angststörungen, Hyperaktivitätsstörung, Schmerzen oder Diabetes.

14. Transdermale Arzneimittelverabreichungsvorrichtung nach einem der Ansprüche 1 bis 9 zur Verwendung beim Behandeln oder Vorbeugen von Schmerzen, wobei der pharmazeutische Wirkstoff ein opioides Analgetikum ist und die Vorrichtung zur Prämedikation eines Patienten vor einer Operation verwendet wird.

15. Verfahren zum Herstellen einer transdermalen Arzneimittelverabreichungsvorrichtung nach einem der Ansprüche 1 bis 11, wobei das Verfahren die folgenden Schritte umfasst:
(a) Erzeugen eines Arzneimittelzuführungselements nach einem der Ansprüche 1 bis 6;
(b) Integrieren des pharmazeutischen Wirkstoffs in oder auf das Arzneimittelzuführungselement; und
(c) Beschichten einer Oberfläche des Arzneimittelzuführungselements mit einem wasserquellbaren Substrat umfassend ein oder mehrere organische Polymere.

## Revendications

1. Dispositif d'administration de médicament transdermique comprenant un élément de délivrance de médicament fixé à un substrat gonflable à l'eau comprenant un ou plusieurs polymères organiques, dans lequel l'élément de délivrance de médicament définit une surface de contact pour placement, en utilisation, contre la peau d'un patient, en outre dans lequel l'élément de délivrance de médicament comprend un ingrédient pharmaceutique actif et un matériau solide poreux, dans lequel le matériau solide poreux est à base d'un ou plusieurs matériaux céramiques ou d'un ou plusieurs matériaux géopolymériques.

2. Dispositif d'administration de médicament transdermique selon la revendication 1, dans lequel le matériau solide poreux est formé à partir d'une céramique autodurcissable.

3. Dispositif d'administration de médicament transdermique selon la revendication 1 ou la revendication 2, dans lequel le matériau solide poreux est à base d'un sulfate de calcium, d'un phosphate de calcium, d'un silicate de calcium, d'un carbonate de calcium ou d'un carbonate de magnésium ; facultativement
dans lequel le matériau solide poreux est du sulfate de calcium hémihydraté ou du phosphate de calcium acide.

4. Dispositif d'administration de médicament transdermique selon l'une quelconque des revendications précédentes, dans lequel :
(a) l'ingrédient pharmaceutique actif est surtout placé au sein des pores du solide poreux ; ou
(b) l'ingrédient pharmaceutique actif est surtout placé sur la surface externe du solide poreux.

5. Dispositif d'administration de médicament transdermique selon l'une quelconque des revendications précédentes, dans lequel l'élément de délivrance de médicament est un réseau de protubérances microscopiques.

6. Dispositif d'administration de médicament transdermique selon l'une quelconque des revendications précédentes, dans lequel la surface de contact de l'élément de délivrance de médicament est moulée pour définir un réseau de protubérances microscopiques ; facultativement dans lequel :
(a) la surface de contact de l'élément de délivrance de médicament est moulée pour définir un réseau de protubérances coniques ou pyramidales microscopiques ; ou
(b) la surface de contact de l'élément de délivrance de médicament est moulée pour définir un réseau de micro-aiguilles.

7. Dispositif d'administration de médicament transdermique selon l'une quelconque des revendications précédentes, dans lequel le substrat gonflable à l'eau est déformable contre la peau d'un patient.

8. Dispositif d'administration de médicament transdermique selon l'une quelconque des revendications 1 à 7, dans lequel les un ou plusieurs polymères organiques sont choisis dans le groupe consistant en la gomme de fenugrec, la gomme de fagotier, la cyclodextrine, le PVA, le caoutchouc de silicium, le poly(méthacrylate de méthyle), le poly(diméthyl siloxane), le poly(éthylène), le poly(propylène), le parylène, la poly(vinylpyrrolidone), le poly(acétate de vinyle), l'alginate (par exemple l'alginate d'ammonium), le chitosan, la gélatine, les copolymères de poly(alcool vinylique), le polyacrylamide, la carboxyméthylcellulose, la poly(vinylimine), le polyacrylate, la gomme de karaya, la copovidone, l'hydroxyéthyl cellulose, l'hydroxypropyl cellulose, la maltodextrine, le poly(oxyde d'éthylène), la poly(vinylamine) et un sel de polyacrylate.

9. Dispositif d'administration de médicament transdermique selon une quelconque revendication précédente, dans lequel l'ingrédient pharmaceutique actif est un antihypertenseur, un sédatif, un hypnotique, un analgésique ou une substance immunogène.

10. Dispositif d'administration de médicament transdermique selon la revendication 9, dans lequel l'ingrédient pharmaceutique actif est choisi parmi la benzodiazépine, la clonidine et le zolpidème, ou un sel pharmaceutiquement acceptable de celui-ci.

11. Dispositif d'administration de médicament transdermique selon l'une quelconque des revendications 1 à 9, dans lequel l'ingrédient pharmaceutique actif est un peptide ou une protéine.

12. Dispositif d'administration de médicament transdermique selon l'une quelconque des revendications précédentes, pour une utilisation en thérapie.

13. Dispositif d'administration de médicament transdermique selon la revendication 9 ou la revendication 10, pour une utilisation dans le traitement de l'insomnie, de l'hypertension, de troubles de l'anxiété, d'un trouble d'hyperactivité, de la douleur ou du diabète.

14. Dispositif d'administration de médicament transdermique selon l'une quelconque des revendications 1 à 9, pour une utilisation dans le traitement ou la prévention de la douleur, dans lequel l'ingrédient pharmaceutique actif est un analgésique opioïde et le dispositif est utilisé en guise de prémédication pour un patient avant chirurgie.

15. Procédé de fabrication d'un dispositif d'administration de médicament transdermique tel que défini dans l'une quelconque des revendications 1 à 11, lequel procédé comprend les étapes de :
(a) préparation d'un élément de délivrance de médicament tel que défini dans l'une quelconque des revendications 1 à 6 ;
(b) incorporation de l'ingrédient pharmaceutique actif dans ou sur l'élément de délivrance de médicament ; et
(c) revêtement d'une surface de l'élément de délivrance de médicament avec un substrat gonflable à l'eau comprenant un ou plusieurs polymères organiques.
